# EUROPÄISCHE PATENTSCHRIFT

(11) **EP 2 559 445 B1**
(45) Veröffentlichungstag und Bekanntmachung des Hinweises auf die Patenterteilung: **12.10.2016**
(21) Anmeldenummer: 12401170.1
(22) Anmeldetag: 10.08.2012
(51) Int. Cl.: A61L 9/00

(54) **Verwendung einer Zusammensetzung zur Neutralisierung von Gerüchen**
Use of a composition for neutralising odours
Utilisation d'une composition destinée à neutraliser les odeurs

(30) Priorität: 17.08.2011 DE 102011052779; 06.03.2012 DE 102012101867
(43) Veröffentlichungstag der Anmeldung: 20.02.2013
(73) Patentinhaber: NODOR GmbH & Co. KG, 28844 Weyhe-Lahausen (DE)
(72) Erfinder: Barnickel, Thomas C., 27283 Verden (DE)
(74) Vertreter: Scheffler, Jörg

(56) Entgegenhaltungen:
- DE-B4-102006 026 305
- FR-A- 996 488

## Beschreibung

Die Erfindung betrifft die Verwendung einer Zusammensetzung zur Geruchsneutralisierung

Die US 2004/0127463 A1 offenbart bereits eine wässrige Zusammensetzung zur Geruchsneutralisierung mit einem Gehalt an 0,01 bis 20 Gewichtsprozent solubilisiertem und komplexiertem Cyclodextrin, optional mit einem Zusatz eines oberflächenaktiven Stoffes und/oder von antimikrobiellen Wirkstoffen, Parfum, Polyol, Komplexbildnern, Metallsalz, vorzugsweise mit einem Zusatz von wasserlöslichen Kupfer- und/oder Zinksalzen, Enzymen oder Konservierungsmitteln. Die geruchsneutralisierende Wirkung der Zusammensetzung wird auf den Gehalt an Cyclodextrin zurückgeführt.

Die WO 01/80914 A1 zitiert die bekannte Verwendung von Triglyceriden und Polyglycosiden zur Verstärkung der geruchsabsorbierenden Wirkung von Polymeren wie Chitosan, Alginaten und synthetischen Polymeren und offenbart überdies die Verwendung kationischer Polysaccharide, insbesondere mit Aminogruppen derivatisierter Polysaccharide, darunter auch Chitosan, zur Verwendung als Geruchsneutralisierer. Daneben wird die Verwendung von Zeolithen als geruchsneutralisierendes Mittel beschrieben. Als weiteres organisches Polymer werden auch derivatisierte Cyclodextrine zur Geruchsneutralisierung verwendet. Die EP 1 070 500 A1 beschreibt ein Desodorant, das neben wasserlöslichen Metallsalzen ein Silikonöl und ein nicht ionisches Detergenz enthält.

Die JP 62-281950 A beschreibt eine Zusammensetzung zur Desodorierung, die Aluminium-, Kupfer-, Zink- und Magnesium-Salze enthält.

Die JP 57-160462 A beschreibt eine Desodorant-Zusammensetzung, die ein Eisen- und Kupfersalz in Wasser enthält, vorzugsweise in Mischung mit Aluminiumsulfat, Aluminiumchlorid, Magnesiumchlorid, Harnstoff und Enzymen.

Die JP 40-26421 A beschreibt eine Zusammensetzung aus Zinksulfat, Natriumchlorid und Kaliumhydrogensulfat, die auch in Lösung oder trocken als Desodorant verwendet werden kann.

Die US 2003/0158297 A1 beschreibt ein Verfahren zur Sprühtrocknung einer organischen Verbindung mit einer keramischen Komponente, beispielsweise kolloidalem Siliziumdioxid.

Die US 2006/0039986 A1 beschreibt die Absorption von Katechin, Vitaminen, Tanninen, Feuchthaltemitteln und pflanzlichen aromatischen Ölen in Verbindung mit einem Beladungsbestandteil an oder in ein organisches Polymer.

Die US 4,501,730 A beschreibt ein desodorierendes Destillat aus der Trockendestillation von Teeblättern.

Die US 6,019,963 A beschreibt eine desodorierende Dispersion mit Teebaum- und Eukalyptusöl.

Die US 1,408,535 A beschreibt eine wasserlösliche Zusammensetzung aus Gips, Formaldehyd, Chlorkalk, Eukalyptusöl, Thymianöl und Lavendelöl.

Die WO 2005/044318 A1 beschreibt die Verdunstung duftender organischer Zusammensetzungen durch ein Kapillarsystem. Die duftenden Inhaltsstoffe sind ausschließlich organische Bestandteile mit einem Molekulargewicht im Bereich von maximal 175 g/M und einer Oberflächenspannung von weniger als 40 Dyn/cm.

Die US 3,317,372 A beschreibt eine desodorierende Zusammensetzung mit einem flüchtigen Material, beispielsweise Ammonium, flüchtigen Aminen und flüchtigen Produkten quartären Ammoniumhydroxids.

Die US 2005/0019269 A1 beschreibt eine basische Zusammensetzung gegen Schimmel, die Thymianöl und optional eine Vielzahl anderer Aromaöle enthält.

Die US 2004/0120851 A1 beschreibt eine biozide Mischung von Partikeln mit einer Größe von 1-50 µm mit einem bioziden Inhaltsstoff, die in Gegenwart von Wasser chemisch reagieren, um biologische Partikel zu beeinflussen. Die Mischung kann beispielsweise Diatomeenerde mit Natriumchlorit und Natriumchlorid sein, gegebenenfalls in Kombination mit Zitronensäure sowie Calciumchlorid. Die chemische Reaktion kann beispielsweise zu Chlordioxid führen.

Die JP 2012-005441 A bezieht sich auf eine Heimtiertoilette, deren Wirkung auf Aluminiumsulfat basiert. Die US 5,741,482 A betrifft eine gelförmige Zusammensetzung zur Luftbehandlung aus einer Vielzahl von möglichen Verbindungen.

Ferner betrifft die DE 10 2006 026 305 B4 eine Zusammensetzung aus Magnesiumsulfat und Aluminiumsulfat zur Neutralisierung von Gerüchen biologischen Ursprungs durch oberflächliches Aufbringen einer wässrigen Lösung der Zusammensetung.

FR996488 beschreibt eine Lösung zur Unterdrückung von Gerüchen bestehend aus: 120g Kaliumaluminiumsulfat, 120g Natriumchlorid, 30g Formaldehyd, 5g Amylacetat als Geruchsstoff, 1 kg Wasser.

Bekannte Zusammensetzungen zur Geruchsneutralisierung enthalten im Allgemeinen organische Polymere, die von Mikroorganismen verstoffwechselt werden können. Daher besteht ein Nachteil dieser bekannten Zusammensetzungen darin, das Wachstum von Mikroorganismen auf den behandelten Oberflächen zu fördern, was wiederum eine Erhöhung der Keimbelastung zur Folge hat. Weiterhin kann die Verstoffwechselung der organischen Polymere, die die geruchsbildenden Stoffe binden, zu deren erneuter Freisetzung führen, sodass die geruchsneutralisierende Wirkung nicht dauerhaft ist.

Gegenüber bekannten Zusammensetzungen zur Geruchsneutralisierung stellt sich der vorliegenden Erfindung die Aufgabe, eine alternative Zusammensetzung zur Geruchsneutralisierung bereitzustellen. Vorzugsweise sollen Zusammensetzungen bereitgestellt werden, die keine schädliche Wirkung auf behandelte Oberflächen ausüben, beispielsweise auf haushaltsübliche Oberflächen aus Kunststoff, Glas, Holz, Keramik oder synthetische bzw. natürliche Textilien einschließlich Teppichen und Leder.

Weiterhin ist es bevorzugt, dass die erfindungsgemäße Zusammensetzung nicht von Mikroorganismen verstoffwechselbar ist, insbesondere keine organischen Bestandteile aufweist. Besonders bevorzugt soll die erfindungsgemäße Zusammensetzung nach der Anwendung als wässrige Lösung und dem Abtrocknen des Lösungswassers keine Spuren auf der behandelten Oberfläche hinterlassen.

Im Sinne der Erfindung wird als Geruchsneutralisierung die Wirkung einer Zusammensetzung so definiert, dass die Abgabe von geruchsbildenden Stoffen aus Geruchsbildnern, beispielsweise biologischen Ursprungs, verhindert wird. Hierbei fallen unter den Begriff der Geruchsbildner menschliche oder tierische Körperausscheidungen wie Kot, Urin und Erbrochenes, wie sie beispielsweise bei der Haltung von Haustieren, bei öffentlichen Toiletten oder bei medizinischen Einrichtungen auftreten. Zu den geruchsbildenden Stoffen zählen beispielsweise Ammoniak, Aldehyde, flüchtige Carbonsäuren und Ester, Thiole, Skatol und andere Verbindungen sowie allgemein Stoffe, die als unangenehmer Geruch wahrnehmbar sind.

Die vorliegende Erfindung betrifft die Verwendung zur Neutralisierung von Gerüchen gemäß Anspruch 1. Die beschriebene Zusammensetzung besteht aus einem wasserlöslichen Natriumchlorid in Mischung mit einem wasserlöslichen Aluminiumsulfat. Die Zusammensetzung wird als wässrige Lösung verwendet, um eine möglichst gleichmäßige oberflächliche Benetzung der Geruchsbildner zu erreichen. Dabei kann die Zusammensetzung als gebrauchsfertige wässrige Formulierung angeboten werden oder als trockene Zusammensetzung, die vor dem Gebrauch durch Zugabe von Wasser in Lösung zu bringen ist.

Dabei wird die beschriebene Zusammensetzung auf die Geruchsbildner aufgebracht, beispielsweise aufgesprüht, aufgegossen oder durch Wischen aufgetragen. Die erfindungsgemäße Zusammensetzung ist eine Feststoffmischung, die in wässriger Lösung anwendbar und daher frei von organischen Lösemitteln sowie chemisch beständig ist und haushaltsübliche Oberflächen, beispielsweise Textilien, Kunststoffe, Glas, Holz oder Leder, nicht angreift.

Die Massenanteile des Aluminiumsulfats bzw. des Natriumchlorids betragen an der Trockensubstanz zwischen 60 und 80 % Aluminiumsulfat und zwischen 20 und 40 % Natriumchlorid, vorzugsweise im Verhältnis von 2:1.

Diese Zusammensetzung aus Natriumchlorid und Aluminiumsulfat ist gegenüber dem Stand der Technik dadurch vorteilhaft, dass diese erfindungsgemäß verwendeten Salze nicht toxisch und nicht umweltgefährdend sind, beispielsweise auch gegen Wasserorganismen nicht toxisch sind.

Erfindungsgemäß liegt der pH-Wert der wässrigen Zusammensetzung im Bereich von 3,5 bis 7, vorzugsweise im Bereich von 4 bis 5, insbesondere bei etwa 4.

Zur Geruchsneutralisierung wird die Zusammensetzung der wässrigen Lösung auf den Geruchsbildner vorzugsweise so aufgesprüht, dass dieser vollständig oberflächlich benetzt ist. Durch den oberflächlichen Auftrag der Zusammensetzung aus wässriger Lösung wird die Abgabe geruchsbildender Stoffe aus dem Geruchsbildner an die Atmosphäre verhindert, sodass auch bei starker Geruchsentwicklung keine Geruchsbelästigung mehr auftritt, was sich sogar für geruchsintensive organische Verbindungen wie Ammoniak feststellen lässt.

Zusätzlich zu dem Gehalt der Zusammensetzung an Natriumchlorid und Aluminiumsulfat, die zur Geruchsneutralisierung wirksam sind, kann eine nicht erfindungsgemäße Zusammensetzung weitere Hilfs- und Duftstoffe enthalten, wie beispielsweise Farbstoffe, Parfüm oder antibakterielle Wirkstoffe, insbesondere quartäre Ammoniumverbindungen.

Als optionaler weiterer Inhaltsstoff kann in einer nicht erfindungsgemäßen Zusammensetzung Teebaumöl und/oder Thymol enthalten sein, die als natürliches Desinfektionsmittel wirken.

Zur Herstellung einer Zusammensetzung wird eine Trockenmischung von Aluminiumsulfat mit Natriumchlorid mit einem Masseverhältnis von 2:1 und einer Dosierung von 32 g/l bei Raumtemperatur in destilliertem Wasser gelöst. Diese Lösung steht dann gebrauchsfertig zur Verwendung.

Die wässrige Zusammensetzung wird auf den Geruchsbildner aufgesprüht, sodass dieser gründlich oberflächlich benetzt ist. Innerhalb sehr kurzer Zeit, spätestens nach 2 bis 5 Minuten, ist der biologische Geruch neutralisiert, d. h. nicht mehr wahrnehmbar.

Diese Wirkung der Zusammensetzung in wässriger Lösung hält auch nach dem Abtrocknen an und ist über einen langen Zeitraum stabil. So konnte beispielsweise in einem praktischen Versuch auch nach zwei bis sechs Monaten kein Wiederauftreten einer Geruchsbelästigung von einer zuvor verunreinigten und daraufhin behandelten Wandoberfläche festgestellt werden.

Wesentliche Vorteile der Zusammensetzung bestehen neben den wesentlich geringeren Beschaffungskosten des Natriumchlorids gegenüber anderen, nach dem Stand der Technik eingesetzten Substanzen vor allem auch darin, dass Natriumchlorid absolut ungiftig ist und keiner CAS- oder CAR-Klassifizierung im chemischen Datenblatt unterliegt.

Zudem führt die vorgesehene Dosierung von 32 g/l Trockensubstanz zu einer wesentlich höheren Ausbeute, nämlich ca. 31 I wässriger Lösung je Kilogramm Trockensubstanz, und ermöglicht daher eine weitere Kostenminderung für das liquide Endprodukt und eine Schonung der Ressourcen.

## Patentansprüche

1. Verwendung einer Zusammensetzung zur Neutralisierung von Gerüchen biologischen Ursprungs durch oberflächliches Aufbringen einer wässrigen Lösung
**dadurch gekennzeichnet, dass** die Zusammensetzung aus einer Lösung von Aluminiumsulfat und Natriumchlorid in Wasser besteht und die wässrige Lösung aus einer Trockenmischung mit einer Dosierung zwischen 20 g/l und 65 g/l, insbesondere zwischen 30 g/l und 35 g/l, zwischen 45 g/l und 50 g/l oder zwischen 60 g/l und 65 g/l, in Wasser hergestellt wird.

2. Verwendung einer Zusammensetzung nach Anspruch 1 oder 2, **gekennzeichnet durch** einen pH-Wert der wässrigen Lösung im Bereich von 3,5 bis 7.

3. Verwendung einer Zusammensetzung nach zumindest einem der vorhergehenden Ansprüche, **gekennzeichnet durch** ein Masseverhältnis in der Trockenmischung von Aluminiumsulfat zu Natriumchlorid im Bereich von 3:1 bis 1:1, insbesondere 2:1.

## Claims

1. Use of a composition for neutralizing odours of biological origin by means of surface application of an aqueous solution, **characterized in that** the composition consists of a solution of aluminium sulphate and sodium chloride in water and the aqueous solution is prepared from a dry mixture with a dosage between 20 g/l and 65 g/l, in particular between 30 g/l and 35 g/l, between 45 g/l and 50 g/l or between 60 g/l and 65 g/l, in water.

2. Use of a composition according to Claim 1, **characterized by** a pH of the aqueous solution in the range from 3.5 to 7.

3. Use of a composition according to at least one of the preceding claims, **characterized by** a mass ratio in the dry mixture of aluminium sulphate to sodium chloride in the range from 3:1 to 1:1, in particular 2:1.

## Revendications

1. Utilisation d'une composition destinée à neutraliser les odeurs d'origine biologique par application superficielle d'une solution aqueuse, **caractérisée en ce que** la composition se compose d'une solution de sulfate d'aluminium et de chlorure de sodium dans de l'eau et la solution aqueuse est préparée à partir d'un mélange sec comprenant un dosage compris entre 20 g/l et 65 g/l, en particulier entre 30 g/l et 35 g/l, entre 45 g/l et 50 g/l ou entre 60 g/l et 65 g/l, dans de l'eau.

2. Utilisation d'une composition selon la revendication 1, **caractérisée par** une valeur pH de la solution aqueuse dans une plage de 3,5 à 7.

3. Utilisation d'une composition selon au moins l'une quelconque des revendications précédentes, **caractérisée par** un rapport de masse dans le mélange sec de sulfate d'aluminium au chlorure de sodium dans une plage de 3:1 à 1:1, en particulier de 2:1.
